Europäisches Patentamt

European Patent Office

Office européen des brevets

(11) Publication number: **0 055 406
B1**

(12) **EUROPEAN PATENT SPECIFICATION**

(45) Date of publication of patent specification: **27.03.85**

(51) Int. Cl.⁴: **C 04 B 35/54, A 61 F 2/24**

(21) Application number: **81110242.5**

(22) Date of filing: **07.12.81**

(54) Method of making all-pyrocarbon prosthetic device components.

(30) Priority: **29.12.80 US 220681**

(43) Date of publication of application:
**07.07.82 Bulletin 82/27**

(45) Publication of the grant of the patent:
**27.03.85 Bulletin 85/13**

(84) Designated Contracting States:
**DE FR GB IT**

(56) References cited:
**FR-A-2 104 563
US-A-3 138 434
US-A-3 399 969
US-A-3 410 746
US-A-3 457 042
US-A-4 178 639
US-A-4 276 132**

(73) Proprietor: **Carbomedics Inc.
1300 East Anderson Lane
Austin Texas (US)**

(72) Inventor: **Bokros, Jack Chester
3117 Ski Terrace Lane
Austin Texas (US)**
Inventor: **Emken, Michael Roy
2836 Cacatua
La Costa California (US)**
Inventor: **Akins, Robert Joe
2509 Scenic Drive
Austin Texas (US)**
Inventor: **Ellis, Willard Harrison
1680 Eolus Avenue
Leucadia California (US)**
Inventor: **Slivenko, Victor
6417 Crawford Street
San Diego California (US)**

(74) Representative: **Weickmann, Heinrich, Dipl.-Ing.
et al
Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)**

Courier Press, Leamington Spa, England.

## Description

### Background of the Invention

Pyrolytic carbon or pyrocarbon is formed by thermally decomposing gaseous hydrocarbons or other carbon-containing substances in vaporous form so that a substrate in the deposition region becomes coated. U.S. Patent No. 3,138,438 shows the formation of a simple anisotropic pyrocarbon article designed for use as a nose cone or the like wherein a mandrel of appropriate shape is coated completely about its exterior surface and subsequently cut in half and separated from the pyrocarbon deposit to create a pair of nose cones. Reentrant angles on the substrate are employed such as to provide for easy separation of the pyrocarbon article from the underlying substrate. U.S. Patent No. 3,399,969 teaches the formation of massive deposits of isotropic pyrolytic carbon. In most instances, these pyrocarbon deposits were intended for permanent use as a part of a composite article, and thus the achievement of a strong bond between the pyrocarbon layer and the underlying substrate was considered to be of significant importance.

Heretofore, objects which were formed from pyrocarbon alone have been limited to relatively simple shapes of an isotropic pyrocarbon, such as tubes, crucibles, nose cones and the like. The simplicity of the shapes has been necessary to facilitate the separation of the underlying substrate from the pyrocarbon deposit without destruction of the relatively thin pyrocarbon object.

### Summary of the Invention

It has now been found that precise complex shapes can be formed totally of pyrolytic carbon to produce all-pyrocarbon objects having advantages over previously employed composite objects of the same shape. Moreover, the formation process allows the formation of machining of the difficult-to-machine pyrocarbon, by initially machining a mirror image of the desired ultimate surface onto the substrate which is relatively easy to machine. Following deposit of pyrocarbon layer of desired thickness, the coated substrate is transferred from the deposition region, and the substrate is subsequently removed from the pyrocarbon layer in a manner which neither chemically nor physically affects the pyrocarbon — for example, by abrading with an appropriate grit.

### Brief Description of the Drawings

FIGURE 1 is a flowsheet illustrating a sequence of steps for forming a pyrocarbon object embodying various features of the invention;

FIGURE 2 is a diagrammatic view of a pyrocarbon deposition chamber suitable for use in the fabrication method illustrated in FIGURE 1;

FIGURE 3 is an elevation view of a substrate which has been carefully machined to have a precise mirror image as it might appear after the first step of the method depicted in FIGURE 1;

FIGURE 4 is a perspective view of the substrate following the step of coating with isotropic pyrocarbon;

FIGURE 5 is a perspective view showing the coated substrate after preliminary removal of pyrocarbon and portions of the substrate;

FIGURE 6 is an enlarged sectional view taken along line 6—6 of FIGURE 5;

FIGURE 7 is a sectional view similar to FIGURE 6 of the annular valve body after the substrate has been removed by abrasion to expose the interior surface of the valve body and finish machining has been carried out; and

FIGURE 8 is a small cross-sectional view of a heart valve showing the final shape of the valve body having the leaflets in place and in the open position.

### Detailed Description of the Preferred Embodiments

In addition to its biocompatibility and thromboresistance, isotropic pyrolytic carbon has the further important characteristic of exhibiting excellent wear resistance. However, this important characteristic of pyrocarbon in an implanted prosthetic device might be considered a disadvantage in its fabrication because pyrocarbon is hard and difficult to machine, and prosthetic devices, particularly heart valves, must be machined to close tolerances to assure their continuing to function in the intended manner over the lifetime of the patient.

In the heart valve prosthesis art, it is also important that the heart valve structure occupies as little space as possible, thus providing the maximum void region for blood flow therethrough consistent with sound structural strength and mechanical functioning of this precisely fashioned device. As disclosed in U.S. Patent No. 4,178,639, issued December 18, 1979 to Jack C. Bokros, it is known to make an annular valve body from a substrate of isotropic graphite, such as that sold under the tradename POCO, which has been suitably coated with pyrocarbon, such as that sold under the trademark PYROLITE. In such a composite object, the strength, biocompatibility and wear resistance exhibited in the heart valve are characteristics of the pyrocarbon coating. Moreover, heart valve bodies which are made in this manner require fairly extensive machining of the as-deposited pyrocarbon surfaces.

Heretofore, heart valves having an annular valve body and one or more relatively movable valve members have been assembled either by stretching the valve body or compressing the valve members. Although graphite substrates covered with Pyrolite are structurally strong and have reasonable flexibility, occasionally breakage may occur during the assembly process. By forming annular valve bodies of substantially totally pyrocarbon, thinner yet strong orifice rings are provided which are inherently more flexible and capable of withstanding greater deformation or stretching without breakage during assembly.

Still another advantage arises from the fact that, when a thin pyrocarbon coating (e.g., 0.15 mm (0.006 inch)) was earlier employed on a substrate, following its precise machining to create the heart valve component, it was necessary to subject the component to X-ray monitoring in order to determine the precise position of the substrate. This enables a determination to be made whether there is the minimum required pyrocarbon thickness present in the final component at specific locations where potential wear will be the greatest. Clearly, this is no longer a problem when the component is manufactured from all pyrocarbon.

It has been found that all of the foregoing disadvantages can be alleviated by forming a prosthetic article entirely from pyrocarbon. Thinner, structurally adequate articles can be made when a substrate is eliminated, and thus the object is formed substantially 100% of the higher strength material. Moreover, it has been found that, by machining a mirror image in the relatively easily machinable substrate material, upon deposition of the pyrocarbon one obtains the precisely proportioned and shaped surface desired for an actual application, for example, as an annular heart valve body.

Isotropic, artificial graphite sold under the tradename POCO serves as an appropriate removable substrate which is relatively easily machinable as compared to pyrocarbon. Accordingly, depending upon the type of object one wishes to make, the mirror image of the more complicated surface is machined in a piece of POCO graphite of appropriate size. Thereafter, the POCO graphite substrate is loaded into a suitable pyrocarbon coating device wherein a layer of pyrocarbon of the desired thickness is deposited as generally taught in U.S. Patent No. 3,399,969, issued September 3, 1968 to Jack C. Bokros et al.

By appropriately shaping the substrate, a heart valve body can be produced which will require only a minimum of further machining. For example, an annular valve body having a complex interior surface and top and bottom faces can be deposited upon a mirror image substrate so that only the least critical outer face need thereafter be machined.

The pyrocarbon which is produced is, of course, very important inasmuch as, without a substrate in the resultant object, it is the sole structural member and must produce the durability and strength needed in the object itself. It is known in the art to control the deposition process by selection of gases, proportion of diluent, selection of temperature, bed size and coating rate, as taught in the aforementioned U.S. patent, to create pyrocarbon having the desired physical characteristics. In this respect, the pyrocarbon deposited should be isotropic in form, having a BAF of not greater than about 1.5, and should have a density of at least 70% of maximum theoretical density. The Bacon Anisotropy Factor (BAF) is an accepted measure of preferred orientation of the layer planes in the carbon structure. The technique of measurement and a complete explanation of the scale of measurement is set forth in an article by G. E. Bacon entitled "A Method for Determining the Degree of Orientation of Graphite" which appeared in the *Journal of Applied Chemistry*, Volume VI, Page 477 (1956).

The isotropic pyrolytic carbon may be deposited having a fairly broad range of apparent crystallite sizes; however, it is preferred that the crystallite size be between about 1 nm (10 Å) and about 10 nm (100 Å). Such pyrocarbon will have a hardness of greater than about 200 (DPH—50 gram load). As also taught in the aforementioned patents, alloying of the pyrocarbon with silicon carbide in a minor amount increases the structural strength without decreasing the biocompatibility of the material, and preferably the pyrocarbon is deposited in a manner so as to contain between about 5 and about 20 weight percent silicon, based upon total amount of silicon plus carbon in the material. As used in this application, the term "pyrocarbon" is intended to include pyrolytic carbon containing a minor percentage of silicon carbide or a similar carbide alloying material.

The deposition process is normally carried out at a temperature between about 1200°C and 1500°C, although higher temperatures could be employed if desired. As well known in the art, the deposition is generally carried out using a mixture of an inert diluent, such as helium or argon, and a carbonaceous gas, usually a hydrocarbon. For example, a suitable coating atmosphere might be about 30 volume percent propane and 70 volume percent argon. On the other hand, instead of propane, a mixture of acetylene and propylene might be used.

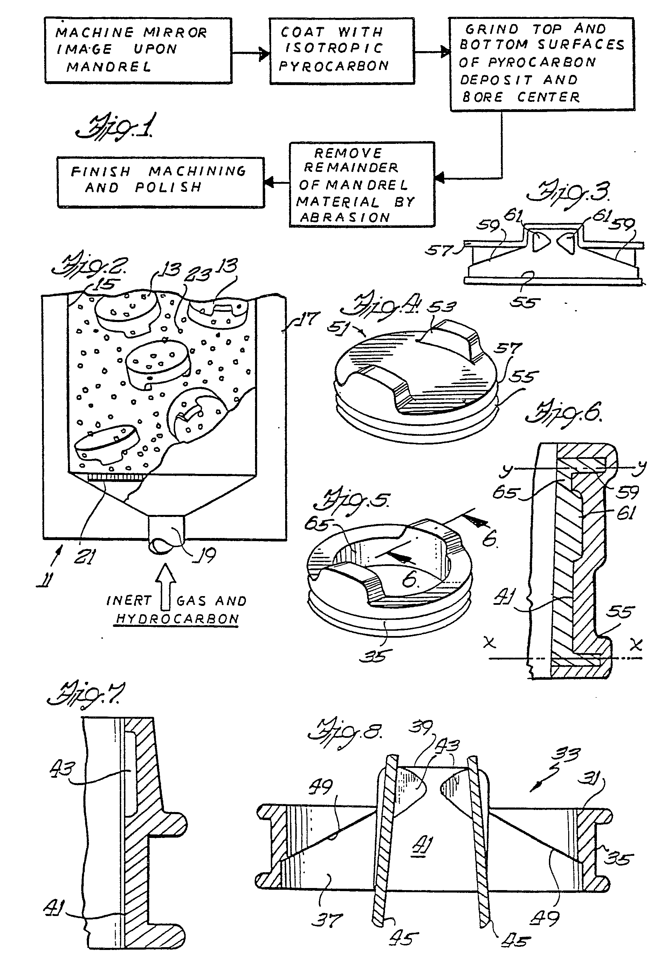

Coating is carried out in a suitable deposition furnace 11 such as that illustrated in FIGURE 2. Articles 13 to be coated are levitated or suspended in an enclosure 15 that is heated by suitable peripheral heater arrangement 17. A fluidizing flow of gas is fed in through a supply conduit 19 at the bottom of the furnace and through a multi-apertured diffuser plate 21 to spread the flow of gas uniformly upward throughout the enclosure. Depending upon the mass of the items and the size of the coater, it may be possible to levitate the items using the gas flow alone. However, the substrates or the articles 13 being coated can be suspended by thin wires or the like from an upper region within the furnace so that they are located within the pyrocarbon deposition region.

As indicated in FIGURE 2, the requisite amount of deposition surface area is provided in the pyrocarbon deposition region by an associated bed 23 of small particles, such as zirconium oxide or silicon carbide particles between about 2 to 500 µm in diameter, as also taught in the aforementioned U.S. patent. The coating conditions are adjusted so as to achieve a satisfactory rate of deposition, i.e. not greater than about 1000 µm per hour, of pyrocarbon which will have the desired physical characteristics previously mentioned.

It is, of course, important that the substrate should have appropriate chemical and physical characteristics so as to render it compatible with these coating conditions and so as to also be relatively easily removable following the completion of the pyrocarbon deposition step. Isotropic artificial graphite is the preferred substrate material, such as that which is sold under the tradename POCO, and preferably the anisotropy of the substrate should measure not greater than about 1.1 BAF. In addition to being stable at the pyrocarbon deposition temperature, the substrate should have a coefficient of thermal expansion (CTE) between about 6 and about $9 \times 10^{-6}°C$. Such a CTE will match or slightly exceed that of the pyrocarbon being deposited so that there should be no difficulty with the possibility of cracking the pyrocarbon during cooling of the coated substrate from the relatively high coating temperature to ambient.

As described in somewhat more detail hereinafter, the deposition of pyrocarbon is carried out for a sufficient length of time to build up a deposit of the desired thickness, which should be at least about 1/4 mm but which will often be greater depending upon the particular object being formed. For example, if an orifice ring (annular valve body) for a heart valve is being formed, the thickness of the pyrocarbon deposit may typically be in the range from about 3/4 mm to about 1.5 mm.

Following deposition and cooling, the excess pyrocarbon which was deposited on the associated surfaces of the substrate may be removed by a rough grinding process leaving a pyrocarbon deposit which resembles that of the ultimate object, and a major portion of the substrate may also be removed, by boring, grinding or the like. The portions of the substrate reasonably distant from the interface where the pyrocarbon deposit began are removable by operations which need not be held to close tolerance. Finally, the remainder of the substrate is removed or eliminated or dissolved using a process that does not chemically or physically affect the pyrocarbon. For example, chemical dissolution of POCO graphite may take place using the mixture of hot silver dichromate and sulfuric acid; alternatively, it can be removed electrolytically in a bath of nitric acid without adversely affecting the pyrocarbon.

Preferably, the pyrocarbon is deposited under conditions such that it has a hardness greater than about 200 as measured on the diamond pyramid hardness (DPH) scale using a 50-gram load. Pyrocarbon of such a hardness will resist abrasion by a suitable grit of substantially lesser hardness (such as sodium bicarbonate or grits of the type used in certain polishing operations which will erode away the remains of the POCO graphite substrate. In fact, the abrasion process actually exerts some polishing effect upon the pyrocarbon which readies its surface for ultimate use as a part of a prosthetic device.

Either prior to, during or following the removal of the remains of the POCO graphite substrate, the pyrocarbon surface of the object that is opposite that which forms adjacent the machined surface of the substrate is finally fashioned or finish-ground. This opposite surface is chosen so that it is a relatively noncritical surface having relatively greater tolerances, e.g. one which may be susceptible to fairly simple semi-automatic grinding. For example, if an orifice ring is being formed by the pyrocarbon deposit, the outer surface is preferably chosen to be the opposite surface which generally requires only the formation of a groove for the accommodation of a suturing ring or the like. By planning so that the more difficult machining is carried out in the easily workable substrate material, such as a relatively soft POCO graphite, it is possible to create a precisely proportioned and shaped interior surface of an orifice ring or the like with only very limited machining of the hard pyrocarbon material.

As an example of one object which may be made in accordance with the invention, FIGURES 3 and 7 illustrate the different stages which may occur in the formation of an orifice ring or valve body 31 of an artificial heart valve 33 of the type shown in cross-section in FIGURE 8. Very briefly, the orifice ring 31 is an annular body having a groove 35 in its outer surface for the acceptance of a suturing ring and having a generally cylindrical inner surface 37 which defines an axial passageway of generally circular cross-section. A pair of standards 39 are formed in upstanding relationship to the major portion of the upper surface of the annular body 31, and each standard includes a flat inner face 41 wherein there are formed a pair of depressions 43 which receive correspondingly shaped ears that protrude from opposite sides of a pair of relatively flat leaflets 45. The depressions 43 are generally triangular or pie-shaped with the lower vertex defining the eccentric pivot axis of one of the leaflets and the upper arcuate edge guiding the pivoting movement of the leaflets 45 between the open and closed portions. To afford a good seat for the arcuate edge of the leaflets 45 in the closed position and to relieve the load from the ears in the closed position, the passageway through the valve body 31 below the leaflets (on the downstream side) is of slightly larger diameter than the passageway above the leaflets (on the upstream side). As a result, a pair of arcuate seats 49 are formed having a downstream facing curved surface which mates with the curved edge of the leaflets.

Accordingly, it can be seen that the interior surface 37 of the valve body 31 requires a fair amount of machining, and in a precision-made device, such as a heart valve for installation in a human patient, very close tolerances must be held. It has been found that, not only can a major portion of the machining of the hard pyrocarbon material be avoided, but a thinner, improved heart valve body can be created by forming the object substantially entirely of pyrocarbon using a method which includes the initial step of appropriately machining the mirror image in a suitable substrate material.

Illustrated in FIGURES 3 and 4 is a short generally cylindrical disc 51 having two upstanding appendages 53 that may be employed to form an orifice ring in accordance with this improved method. The outer surface of the cylinder 51 will constitute the deposition surface for formation of the interior surface at the orifice ring. The flat bottom surface of the orifice ring is machined into the cylinder as a flat flange 55 at the bottom of a groove extending 360° about the surface. The top surface is formed by a similar flange 57 which is interrupted in the regions of the two appendages 53 where the standards are to be formed. The mirror image of the interior surface 37 of the valve body is also machined in the outer surface of the disc 51 which includes the counterparts 59 of the downstream-facing arcuate ledges 49 serving as the seats for each of the leaflets in the closed position and the flat faces 41. The depressions 43 in the flat faces take the form of pie-shaped projections 61 which extend outward from the flat diametrically opposed surfaces.

This relatively small object of machined POCO graphite, which may be about 22 to 25 mm in diameter and about 4 to 5 mm high in the region other than that of the standards, is coated with pyrocarbon in a suitable coating furnace for a time sufficient to create a deposit of pyrocarbon having an average thickness of about 1 mm. At a deposition temperature of about 1400°C, and using a mixture of about 30 percent propane and 70 percent argon, the pyrocarbon deposited will have a density of about 1.8 grams/cm$^3$. Preferably the pyrocarbon is alloyed with silicon carbide, which increases its strength and hardness, and methyltrichlorosilane may be added to the gaseous mixture to produce such alloyed pyrocarbon having a density of about 2 g/cm$^3$ and having a hardness just above 250 (DPH—50 gram load). The presence of the bed of small particles assures a highly isotropic pyrocarbon, i.e. having an anisotropy of not greater than 1.1 BAF. At a total flow rate of about 20 liters per minute (S.T.P.), a coating rate of about 5 microns per minute is achieved so that the total time of deposition may be about 200 minutes. The pyrocarbon-coated substrate may have the general appearance shown in FIGURE 4 with the outer surface being completely enveloped in dense pyrocarbon.

Following the cooling of the coated substrate to ambient conditions, the center of the substrate can be bored out leaving a thin layer 65, i.e. about a millimeter thick of the graphite substrate interior of the diametrically opposed flat surfaces of the standards as illustrated in FIGURES 5 and 6. Likewise, the pyrocarbon and graphite lying more than one-quarter millimeter below the bottom surface can be removed, as by grinding to about the line x—x in FIGURE 6. The region above the upper surface and also above the standards (as indicated by line y—y) can also be removed. The composite object, as illustrated in FIGURE 5, has a natural configuration in its outer surface, generally as a result of the location of top and bottom flanges 57, 55, of a peripheral groove in the region where the groove 35 of the suture ring is desired, thus minimizing the amount of machining necessary to finish the groove.

Following the boring and the top and bottom grinding operations, the remainder of the graphite substrate, which is now totally exposed, is removed by transferring the object to a device for the sand-blasting or abrading of objects, using a suitable grit such as sodium bicarbonate, which has a hardness substantially less than the hardness of the pyrocarbon. The removal of the remainder of the original graphite substrate by the grit also effects a prepolishing of the surfaces upon which the pyrocarbon was deposited, and the complex interior surface and the upper and lower surfaces of the valve body 31 are essentially ready for a finish polishing operation. Minimal machining is necessary along the circumference of the upper and lower edges of the orifice ring and in the region of the groove for the suture ring, and upon its completion and finish polishing, the fabrication of the orifice ring 31 is complete.

The resultant all-pyrocarbon orifice ring 31 is substantially thinner in profile than one made from a composite of a substrate plus pyrocarbon, and accordingly a slightly larger central flow passageway is provided in a heart valve 33 of a given exterior diameter which reduces the overall resistance to the flow of blood therethrough. Moreover, the all-pyrocarbon nature provides elasticity greater than that of a composite object and thus allows for easier installation of the leaflets 45 into the depressions 43 in the standards, and it also permits greater flexibility of design of mating components in prosthetic devices in general. It of course eliminates substantially all of the precision grinding of pyrocarbon and obviates the need for X-ray monitoring to assure minimum pyrocarbon thicknesses are present at certain locations on the orifice ring.

Although the invention has been described with regard to a preferred embodiment which constitutes the best mode known to the inventors, it should be understood that various modifications and changes may be made without departing from the scope of the invention which is defined by the claims appended hereto. For example, it might be desirable to carry out some of the machining steps on the pyrocarbon outer surfaces prior to the elimination of all, or even part of, the graphite substrate material. Likewise, if the exterior shape of an object (as opposed to interior surface of an annular heart valve body) is the more complex one from a machining standpoint, the mirror image of the outer surface may be machined on the interior surface of an annular substrate instead of on the exterior surface of a cylindrical substrate. Of course, the invention is not restricted to the making of heart valve bodies, but other prosthetic device components of relatively complex shape, such as curved and flat heart valve occluders, components for percutaneous implants and artificial joints may likewise be advantageously fabricated.

Various features of the invention are emphasized in the claims which follow.

## Claims

1. A method of making an orifice ring for an artificial heart valve substantially entirely of pyrocarbon having an interior surface of precise shape and dimensions, which method comprises forming a substrate having an arcuate surface, which extends for 360° and is the mirror-image of the desired orifice ring interior surface, from a material that is stable at pyrolytic decomposition temperatures, heating said substrate to a desired temperature in a zone containing a fluidized bed of small particles through which a carbonaceous atmosphere is passed under conditions which cause the deposition of substantially isotropic pyrocarbon for a length of time sufficient to coat said substrate with a layer of pyrocarbon at least about 0.25 mm thick, withdrawing said coated substrate from said deposition zone, and removing said substrate material in a manner which does not physically or chemically affect said pyrocarbon to leave a pyrocarbon ring-like object having an interior surface which is the mirror-image of said substrate surface.

2. A method in accordance with Claim 1 wherein said removal process includes the step of abrading with grit having a hardness less than hardness of said pyrocarbon.

3. A method of making an object having a pyrocarbon surface of precise shape and dimensions, which method comprises forming a substrate having a precisely proportioned surface which is the mirror-image of the desired surface of said object from a material that is stable at pyrolytic decomposition temperatures and that is less resistant to abrasion than pyrocarbon, heating said substrate to a desired temperature in a zone containing a fluidized bed of small particles through which a carbonaceous atmosphere is passed under conditions which cause the deposition of pyrocarbon for a length of time sufficient to coat said substrate with a layer of pyrocarbon of a thickness sufficient to constitute a self-supporting object, said layer being at least about 1/4 mm thick, withdrawing said coated substrate from said deposition zone, and removing said substrate material by abrading with grit which erodes said substrate material without physically affecting said pyrocarbon to leave a pyrocarbon surface which is the mirror-image of said precisely proportioned substrate surface.

4. A method of making an object in accordance with Claim 3 wherein said pyrocarbon has a density of at least 70% of maximum theoretical density.

5. A method of making an object in accordance with Claim 4 wherein said pyrocarbon contains between about 5 and about 20 weight percent silicon, based upon total amount of silicon and carbon in said layer.

6. A method of making an object in accordance with Claim 4 wherein said pyrocarbon has a hardness greater than about 250 (DPH—50 gram load).

7. A method in accordance with Claim 3 wherein said substrate material is stable up to at least about 1500°C in said coating atmosphere and has a C.T.E. of between about 6 and about $9 \times 10^{-6}$/°C.

8. A method in accordance with Claim 7 wherein said substrate material is artificial graphite having an anisotropy of not greater than about 1.1 BAF and said pyrocarbon has a BAF of not greater than about 1.5.

9. A method in accordance with Claim 3 wherein said pyrocarbon is deposited from an atmosphere containing in inert diluent gas and a hydrocarbon at a temperature of between 1200°C and about 1500°C and has a BAF of not greater than about 1.5.

10. A method in accordance with Claim 9 wherein said atmosphere contains at least about 30 volume percent propane and wherein said deposition is effected at a rate not greater than about 1000 µm per hour.

## Revendications

1. Procédé de fabrication d'une bague d'ouverture d'une valvule cardiaque artificielle, en presque totalité en graphite pyrolytique, possédant une surface intérieure à forme et dimensions précises, procédé consistant à former un substrat possédant une surface incurvée qui s'étend sur 360° et est l'image spéculaire de la surface intérieure de la bague d'ouverture voulue, en un matériau qui est stable aux températures de décomposition pyrolytique, à chauffer ledit substrat à une température voulue dans une zone contenant un lit fluidisé de petites particules à travers lequel on fait passer une atmosphère charbonneuse dans des conditions qui provoquent la déposition d'un graphite pyrolytique essentiellement isotrope pendant une période de temps suffisante pour revêtir ledit substrat d'une couche de carbone pyrolytique d'au moins environ 0,25 mm d'épaisseur, à enlever ledit substrat revêtu de ladite zone de déposition, et à enlever ledit matériau de substrat d'une manière qui n'affecte pas, physiquement ou chimiquement, ledit graphite pyrolytique pour laisser un objet en graphite pyrolytique, en forme de bague, ayant une surface intérieure qui est l'image spéculaire de la surface dudit substrat.

2. Procédé selon la revendication 1, dans lequel ladite opération d'enlèvement comprend une étape d'abrasion avec un abrasif ayant une dureté inférieure à la dureté dudit graphite pyrolytique.

3. Procédé de fabrication d'un objet ayant une surface, en graphite pyrolytique, de forme et de dimensions précises, procédé consistant à former un substrat ayant une surface dont les dimensions sont déterminées avec précision, qui est l'image spéculaire de la surface voulue dudit objet, en un matériau qui est stable aux températures de décomposition pyrolytique et qui est moins résistant à l'abrasion que le graphite pyrolytique, à chauffer ledit substrat à une température voulue dans une zone contenant un lit fluidisé de petites particules à travers lequel on fait passer une atmosphère charbonneuse dans des conditons qui provoquent la décomposition du graphite pyrolytique pendant une période de temps suffisante pour revêtir ledit substrat d'une couche de graphite pyrolytique ayant une épaisseur suffisante pour constituer un objet autoporteur, ladite couche ayant une épaisseur d'au moins environ 1/4 mm, à enlever ledit substrat enrobé de ladite zone de déposition et à enlever le matériau dudit substrat par abrasion avec un abrasif qui érode le matériau dudit substrat sans affecter physiquement ledit graphite pyrolytique pour laisser une surface de graphite pyrolytique qui est l'image spéculaire de la surface dudit substrat à dimensions déterminées avec précision.

4. Procédé de fabrication d'un objet selon la revendication 3, dans lequel ledit graphite pyrolytique présente une densité d'au moins 70% de la densité maximale théorique.

5. Procédé de fabrication d'un objet selon la revendication 4, dans lequel ledit graphite pyrolytique contient entre environ 5 et environ 20% en poids de silicium, sur la base de la quantité totale de silicium et de carbone dans ladite couche.

6. Procédé de fabrication d'un objet selon la revendication 4, dans lequel ledit graphite pyrolytique présente une dureté supérieure à environ 250 (essai de dureté au cône de diamant, charge 50 g).

7. Procédé selon la revendication 3, dans lequel le matériau dudit substrat est stable jusqu'à au moins environ 1500°C dans ladite atmosphère de revêtement et présente un coefficient de dilatation thermique compris entre environ 6 et environ 9 $10^{-6}$/°C.

8. Procédé selon la revendication 7, dans lequel le matériau dudit substrat est un graphite artificiel présentant une anisotropie non-supérieure à environ 1,1 BAF (facteur d'anisotropie de Bacon), ledit graphite pyrolytique présentant un facteur d'anisotropie de Bacon BAF non-supérieur à environ 1,5.

9. Procédé selon la revendication 3, dans lequel ledit graphite pyrolytique est déposé à partir d'une atmosphère contenant un gaz diluant inerte et un hydrocarbure à une température comprise entre environ 1200°C et environ 1500°C, et possède un facteur d'anisotropie de Bacon non-supérieur à environ 1,5.

10. Procédé selon la revendication 9, dans lequel ladite atmosphère contient au moins environ 30% en volume de propane, et dans lequel ladite déposition est effectuée à une vitesse non-supérieure à environ 1000 μm par heure.

**Patentansprüche**

1. Verfahren zur Herstellung eines Öffnungsrings für ein künstliches Herzventil, der im wesentlichen vollständig aus Pyrokohlenstoff besteht und eine innere Oberfläche von genauer Gestalt und Abmessungen besitzt, dadurch gekennzeichnet, daß man ein Substrat mit einer gekrümmten Oberfläche, die sich über 360° erstreckt und das Spiegelbild der inneren Oberfläche des gewüngschten Öffnungsrings ist, aus einem Material bildet, das bei pyrolytischen Zersetzungstemperaturen stabil ist, das Substrat auf eine gewünschte Temperatur in einer Zone erhitzt, die ein Fließbett aus kleinen Teilchen enthält, durch welches eine kohlenstoffhaltige Atmosphäre unter Bedingungen geführt wird, die die Abscheidung von im wesentlichen istotropem Pyrokohlenstoff während eines Zeitraums verursachen, der ausreicht, um das Substrat mit einer Schicht von Pyrokohlenstoff von mindestens 0,25 mm Dicke zu beschichten, das beschichtete Substrat aus der Abscheidungszone entfernt, und das Substratmaterial in einer Weise entfernt, die den Pyrokohlenstoff nicht physikalisch oder chemisch beeinflußt, um einen ringförmigen Gegenstand aus Pyrokohlenstoff zu hinterlassen, der eine innere Oberfläche besitzt, die das Spiegelbild der Substratoberfläche ist.

2. Verfahren nach Anspruch 1, dadurch gekennzeichnet, daß das Verfahren zur Entfernung die Stufe des Abschleifens mit grobem Sand enthält, der eine Härte besitzt, die geringer als die Härte des Pyrokohlenstoffs ist.

3. Verfahren zur Herstellung eines Gegenstands mit einer Oberfläche aus Pyrokohlenstoff von genauer Gestalt und Abmessungen, dadurch gekennzeichnet, daß man ein Substrat mit einer genau bemessenen Oberfläche, die das Spiegelbild der gewünschten Oberfläche des Gegenstands ist, aus einem Material bildet, das bei pyrolytischen Zersetzungstemperaturen stabil ist und gegen Abrieb weniger widerstandsfähig als Pyrokohlenstoff ist, das Substrat auf eine gewünschte Temperatur in einer Zone erhitzt, die ein Fließbett aus kleinen Teilchen enthält, durch welches eine kohlenstoffhaltige Atmosphäre unter Bedingungen geführt wird, die die Abscheidung von Pyrokohlenstoff während eines Zeitraums verursachen, der ausreicht, um das Substrat mit einer Schicht von Pyrokohlenstoff zu beschichten, die eine Dikke besitzt, die ausreicht, um einen selbsttragenden Gegenstand zu bilden, wobei diese Schicht mindestens etwa 1/4 mm dick ist, das beschichtete Substrat aus der Abscheidungszone entfernt, und das Substratmaterial durch Abschleifen mit grobem Sand entfernt, der das Substratmaterial erodiert, ohne den Pyrokohlenstoff physikalisch anzugreifen, um eine Oberfläche aus Pyrokohlenstoff zu hinterlassen, die das Spiegelbild der genau bemessenen Substratoberfläche ist.

4. Verfahren zur Herstellung eines Gegenstands gemäß Anspruch 3, dadurch gekennzeichnet, daß der Pyrokohlenstoff eine Dichte von mindestens 70 % der maximalen theoretischen Dichte besitzt.

5. Verfahren zur Herstellung eines Gegenstands gemäß Anspruch 4, dadurch gekennzeichnet, daß der Pyrokohlenstoff zwischen etwa 5 und etwa 20 Gew.-% Silicium enthält, bezogen auf die Gesamtmenge an Silicium und Kohlenstoff in dieser Schicht.

6. Verfahren zur Herstellung eines Gegenstands gemäß Anspruch 4, dadurch gekennzeichnet, daß der Pyrokohlenstoff eine Härte größer als etwa 250 (DPH-50 g Belastung) besitzt.

7. Verfahren nach Anspruch 3, dadurch gekennzeichnet, daß das Substratmaterial in der Beschichtungsatmosphäre bis zu mindestens etwa 1500°C stabil ist und einen C.T.E. (thermischen Ausdehungskoeffizienten) zwischen etwa 6 und etwa $9 \times 10^{-6}$/°C besitzt.

8. Verfahren nach Anspruch 7, dadurch gekennzeichnet, daß das Substratmaterial künstlicher Graphit ist, der eine Anisotropie besitzt, die nicht größer als etwa 1,1 BAF ist und der Pyrokohlenstoff eine BAF besitzt, die nicht größer als etwa 1,5 ist.

9. Verfahren nach Anspruch 3, dadurch gekennzeichnet, daß der Pyrokohlenstoff aus einer Atmosphäre, die ein inertes Verdünnungsgas und einen Kohlenwasserstoff enthält, bei Temperaturen zwischen etwa 1200°C und etwa 1500°C abgeschieden wird und eine BAF besitzt, die nicht größer als etwa 1,5 ist.

10. Verfahren nach Anspruch 9, dadurch gekennzeichnet, daß die Atmosphäre mindestens etwa 30 Vol.-% Propan enthält und worin die Abscheidung mit einer Geschwindigkeit bewirkt wird, die nicht größer als etwa 1000 µm pro Stunde ist.

Fig. 1

MACHINE MIRROR IMAGE UPON MANDREL → COAT WITH ISOTROPIC PYROCARBON → GRIND TOP AND BOTTOM SURFACES OF PYROCARBON DEPOSIT AND BORE CENTER

FINISH MACHINING AND POLISH ← REMOVE REMAINDER OF MANDREL MATERIAL BY ABRASION

Fig. 2

Fig. 3

Fig. 4

Fig. 5

Fig. 6

Fig. 7

Fig. 8

INERT GAS AND HYDROCARBON

1